# EUROPEAN PATENT SPECIFICATION

(11) **EP 2 586 411 B1**
(45) Date of publication and mention of the grant of the patent: **06.08.2014**
(21) Application number: 12007336.6
(22) Date of filing: 25.10.2012
(51) Int. Cl.: A61F 13/15

(54) **Method and apparatus for applying trapezoidal elastic wings to the chassis of diaper**
Verfahren und Vorrichtung zum Aufbringen trapezförmiger elastischer Flügel am Körper einer Windel
Procédé et appareil permettant d'appliquer des ailes élastiques trapézoïdales sur le châssis de la couche-culotte

(30) Priority: 27.10.2011 IT MI20111956
(43) Date of publication of application: 01.05.2013
(73) Proprietor: G.F.A. di Mandotti Angelo & C., 26020 Palazzo Pignano (CR) (IT)
(72) Inventor: Borgo, Giovanni, 26020 Palazzo Pignano (Cremona) (IT)
(74) Representative: Forattini, Amelia

(56) References cited:
- WO-A2-2008/155618
- US-A1- 2008 262 461

## Description

The present invention relates to a method and an apparatus for applying trapezoidal elastic wings to the chassis of a diaper.

As is known, a diaper is constituted generally by a chassis, which is formed by an absorbent material comprised between two sheets, known as topsheet and backsheet, to which side panels are applied which allow to fasten the diaper when it is worn.

The side panels have a flap, which is adhesive or provided with Velcro, and is called tape. The tape is used to fasten the side panel to the chassis.

Figure 17 is a schematic view of the diaper structure described above, generally designated by the reference numeral 100 and constituted by a chassis 102 having two side panels 103 provided with a tape 104.

Figure 18 shows a diaper 101 provided with front panels 105.

The tape is an element made of elastic material, plastic material or fabric, which keeps the diaper closed, when it is worn, by virtue of its adhesive or Velcro parts.

The panel is generally made of elastic fabric in order to allow to increase the fit of the diaper when it is worn. The raw material used for the panel is relatively expensive and affects considerably the overall production cost of the diaper.

The panel and tape assembly forms an elastic wing that is applied to the chassis of the diaper.

Various systems for applying the elastic wings to the chassis are known.

Some manufacturers apply the tapes 104 to the panels 103, which have a rectangular shape, by means of a process that provides two independent and separate strips of material.

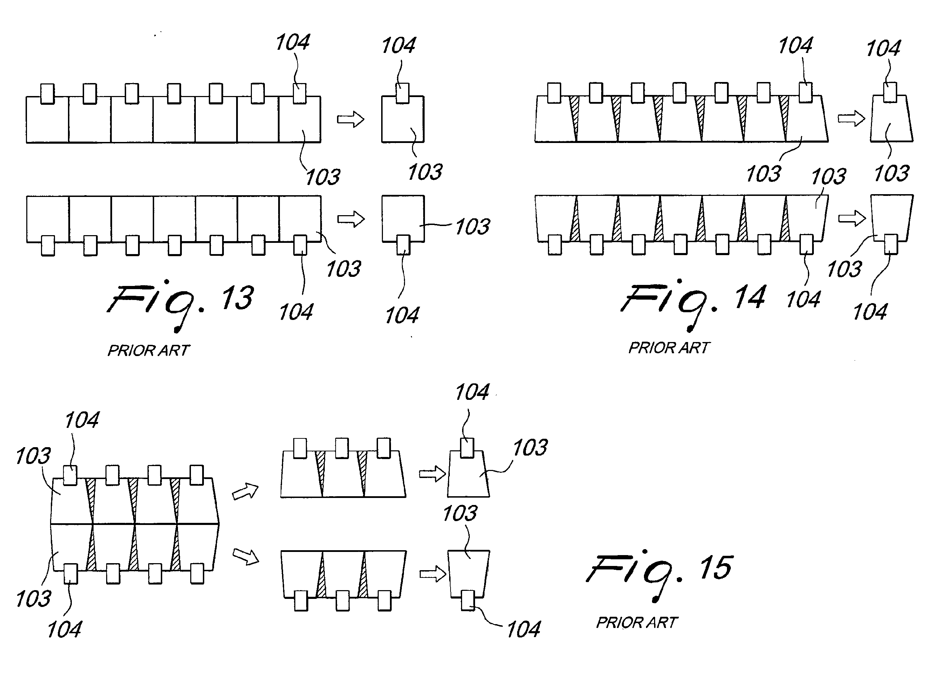

In such system, shown schematically in Figure 13, the elastic wing is formed by functional units, adapted to apply the tape to the panel, and separate unwinders for the panel and for the tape: a set of such separate units for the right hand side and a set of separate units for the left hand side of the diaper.

The elastic wing is applied to the chassis by a single functional unit that applies the wing to both sides of the chassis.

Such system offers the advantage of a certain simplicity of the process, ease of optimization, no waste of raw material, and a low operating cost of the process.

However, the system described above has the severe drawbacks of using a large quantity of raw material because of the size of the panel, having an unsatisfactory aesthetic appearance of the wing, and the fact that the functional unit for the application of the elastic wing is constrained to a particular size of the diaper manufactured in the machine.

Another known production method, shown schematically in Figure 14, provides for the use of a trapezoidal panel with separate wing forming lines.

According to such system, the tapes 104 are applied to panels 103 that have a trapezoidal shape, by means of two independent and separate strips of material.

The elastic wing is made by functional units that form the contour of the panel and apply the tape to the panel. The functional units also comprise separate unwinders for the panel and the tape. There is a set of functional units for the right hand side and a set of functional units for the left hand side, of the diaper.

The elastic wing is applied to the chassis by means of a single functional unit that applies it on both sides.

Such system offers the advantage of an improved aesthetic appearance of the wing, because the trapezoidal shape is perceived by the consumer as more advantageous as regards the fit of the diaper.

However, the system described above has the severe drawback of generating a considerable waste of raw material, due to the triangular portions that are discarded after cutting the panel.

Moreover, such system constrains the functional unit for application of the elastic wing to the size of the diaper manufactured in the machine.

The above system is expensive.

A third production system, shown schematically in Figure 15, provides for the application of the tape 104 to trapezoidal panels 103, by means a process that uses a single strip of material.

Such system has a single unwinder for the panels, two unwinders for the tapes, a single functional unit for shaping the panel and a single functional unit for applying the tape.

Such system forces to use double-width raw material.

The elastic wings are separated longitudinally and spaced transversely only later.

The elastic wing is applied to the chassis by a single functional unit, which applies the wings to both sides.

Such system offers the aesthetic advantage of the trapezoidal wing but a reduced production cost with respect to the system with trapezoidal panel and separate wing forming lines.

However, the system of Figure 15 still has the disadvantage of the waste of raw material and also the raw material has a double width.

Another disadvantage is constituted by the fact that the functional unit for the application of the elastic wing is constrained to the size of the diaper manufactured in the machine.

Another conventional production system provides for the application of the tapes to trapezoidal panels by means of a strip of material that is cut longitudinally, and spaced, and wherein alternately inverted elastic wings are provided.

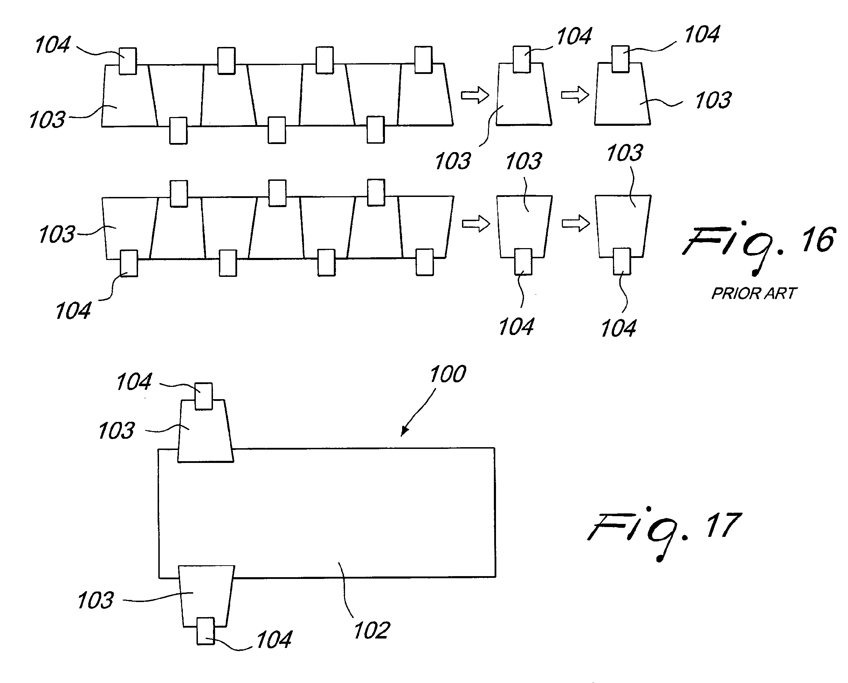

Such system, shown schematically in Figure 16, has functional units for shaping the panels and for the application of the tapes, and separate unwinders for the panels and tapes: a set of such units for the right hand side and a set of units for the left hand side of the diaper. The elastic wings are obtained without waste of material but they have to be alternately rotated prior to their application.

Each elastic wing is applied to the chassis by a single functional unit that applies it on both sides.

Such system allows to obtain a trapezoidal elastic wing without waste of raw material but has a considerable technological complexity.

The system has difficulties in optimization, constrains the functional unit for the application of the elastic wings to the diaper size manufactured in the machine, and is expensive from the production standpoint.

US2008/0262461 discloses a method of making diaper side panels from a continuous strip and applying said side panels to a chassis, according to the conventional systems described above.

The aim of the present invention is to provide a method and an apparatus for applying trapezoidal elastic wings to the chassis of a diaper that overcome the drawbacks of the cited prior art.

Within the scope of this aim, an object of the invention is to provide a method and an apparatus that allow to reduce the number of components with respect to machines having a similar performance.

Another object of the invention is to provide a method and an apparatus that are capable of eliminating waste of expensive raw material.

Another object is to provide a method and an apparatus that allow to use the same functional unit for the application of the elastic wing for any diaper size.

Another object of the invention is to allow to provide a product with first-rate functional and aesthetic characteristics.

Another important object of the invention is to provide a method and an apparatus that are simple and easy to optimize.

This aim and these and other objects which will become better apparent hereinafter are achieved by a method for applying trapezoidal elastic wings to the chassis of a diaper, **characterized in that** it comprises the steps of:
providing a panels ribbon, from which alternating adjacent trapezoidal panels are obtained, each having a smaller base and a larger base that are alternated on each side of said panels ribbon;
applying a tape to said panels ribbon, at the smaller base of each of said panels, and providing an elastic wing constituted by said trapezoidal panel provided with said respective tape;
applying said wings to a first side of a support material ribbon, by means of a single functional unit;
diverting said support material ribbon and feeding again said support material ribbon to said single functional unit;
applying said wings to a second side of said support material ribbon by means of said single functional unit.

This aim and these objects are also achieved by an apparatus for applying trapezoidal elastic wings to the chassis of a diaper, **characterized in that** its comprises a single functional unit constituted by a shaping device and by a pair of wing applicators; said single functional unit receiving a support material ribbon and a panels ribbon; said support material ribbon having a first side and a second side; said panels ribbon having first and second elastic wings provided thereon; said single functional unit applying first elastic wings to a first side of said support material ribbon; said apparatus comprising an axial diversion means; said axial diversion means receiving said support material ribbon, provided with first wings applied to said first side, and feeding said support material ribbon, provided with first wings applied to said first side, to said single functional unit; said single functional unit applying said second wings to said second side of said support material ribbon, aligned with said first elastic wings.

Further characteristics and advantages will become better apparent from the description of preferred but not exclusive embodiments of the invention, illustrated by way of non-limiting example in the accompanying drawings, wherein:

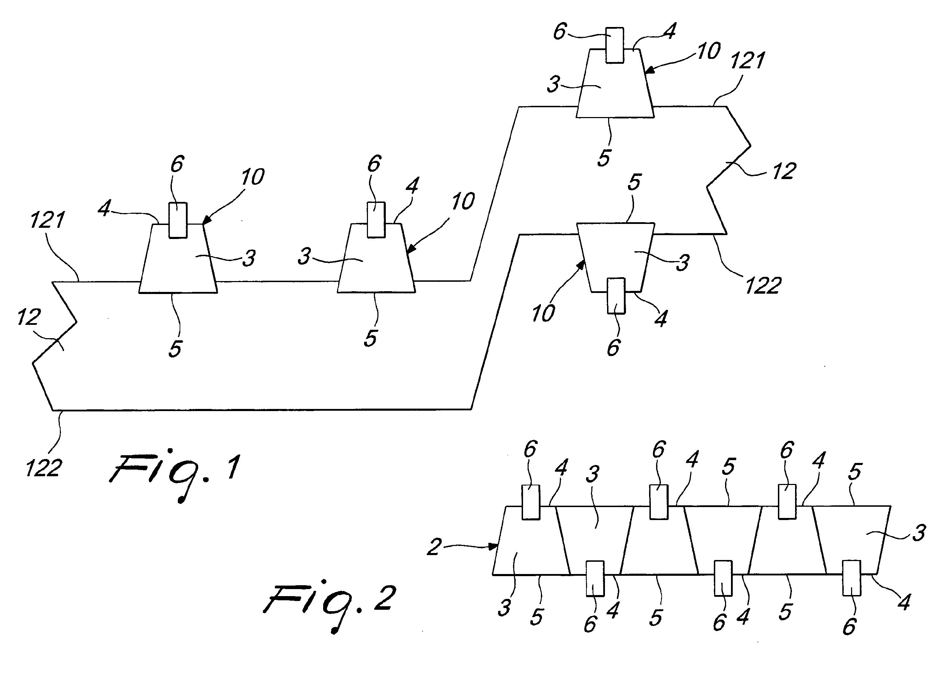

Figure 1 is a schematic view of a portion of supporting fabric with wings applied to one side and, after its diversion, also to the other side, according to the method of the present invention;
Figure 2 is a schematic view of a portion of ribbon of elastic material for forming elastic wings, according to the present invention;

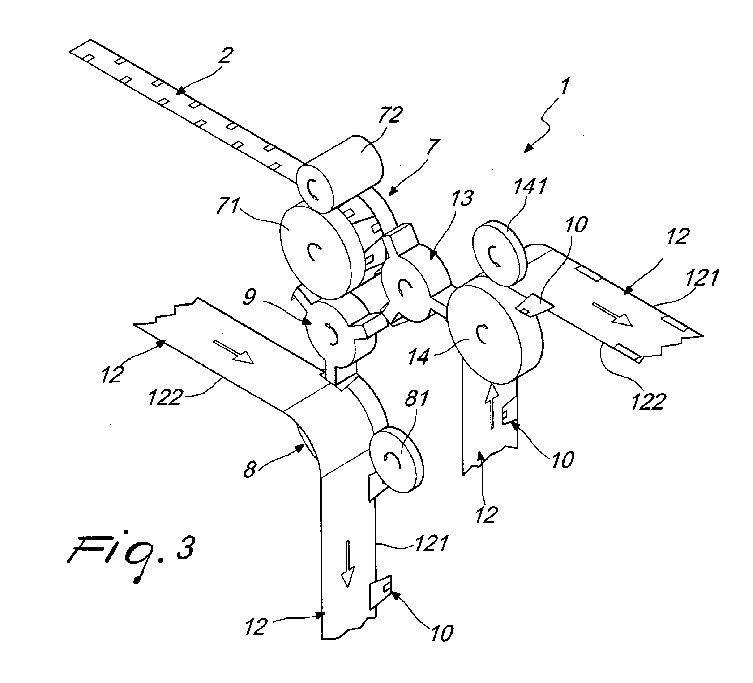

Figure 3 is a schematic perspective view of the essential components of the apparatus according to the present invention;

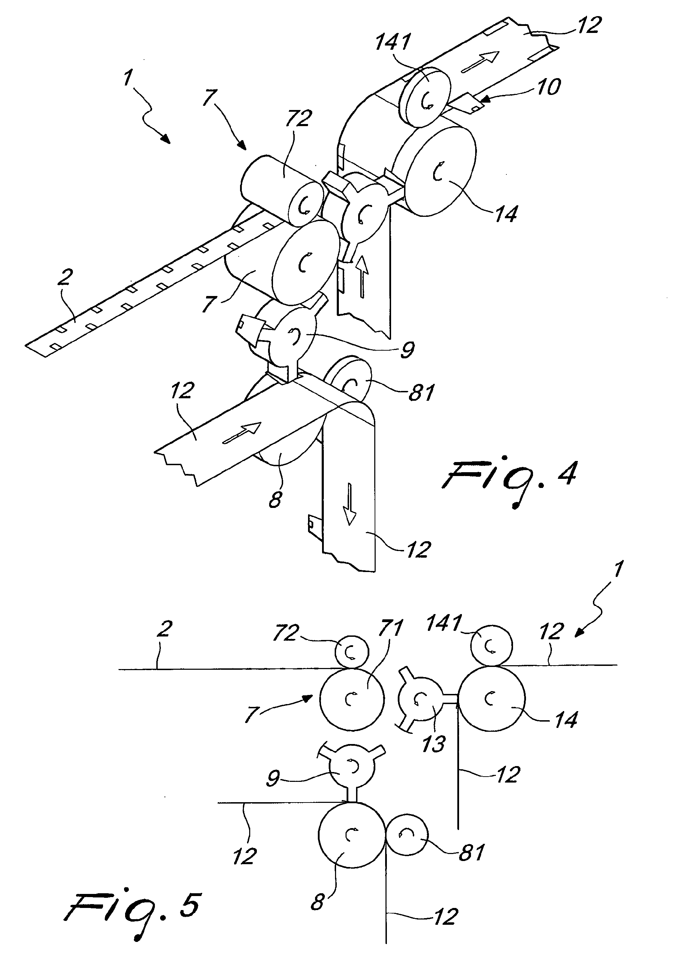

Figure 4 is a perspective view, taken from the opposite side with respect to the preceding view, of the apparatus;
Figure 5 is a diagram of the apparatus of the preceding figure;

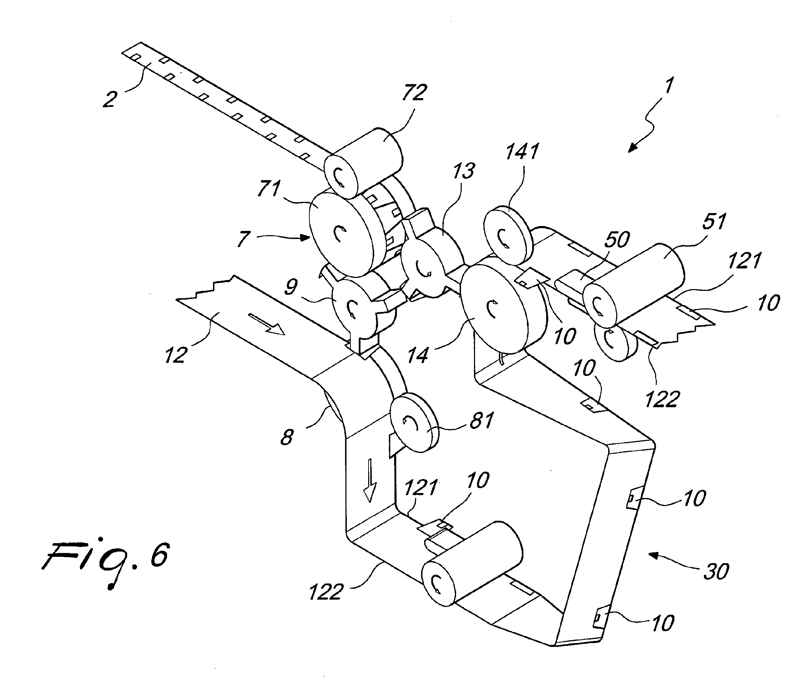

Figure 6 is a schematic perspective view of the apparatus, including a binding means for binding the folded wings, according to the present invention;

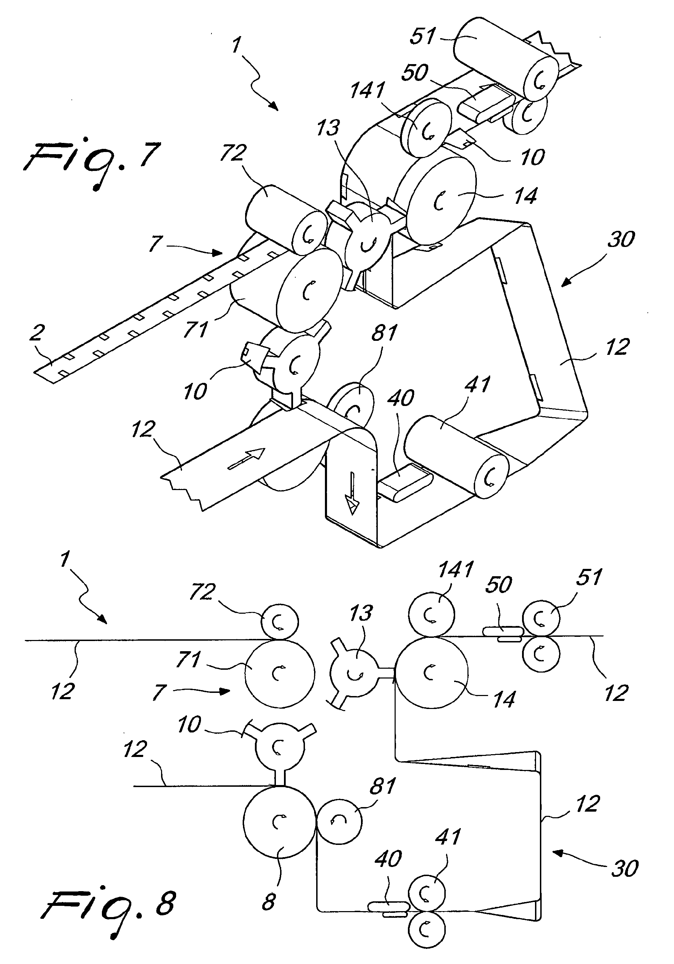

Figure 7 is a perspective view, from the opposite side with respect to the preceding figure, of the apparatus;
Figure 8 is a diagram of the apparatus of the preceding figure;

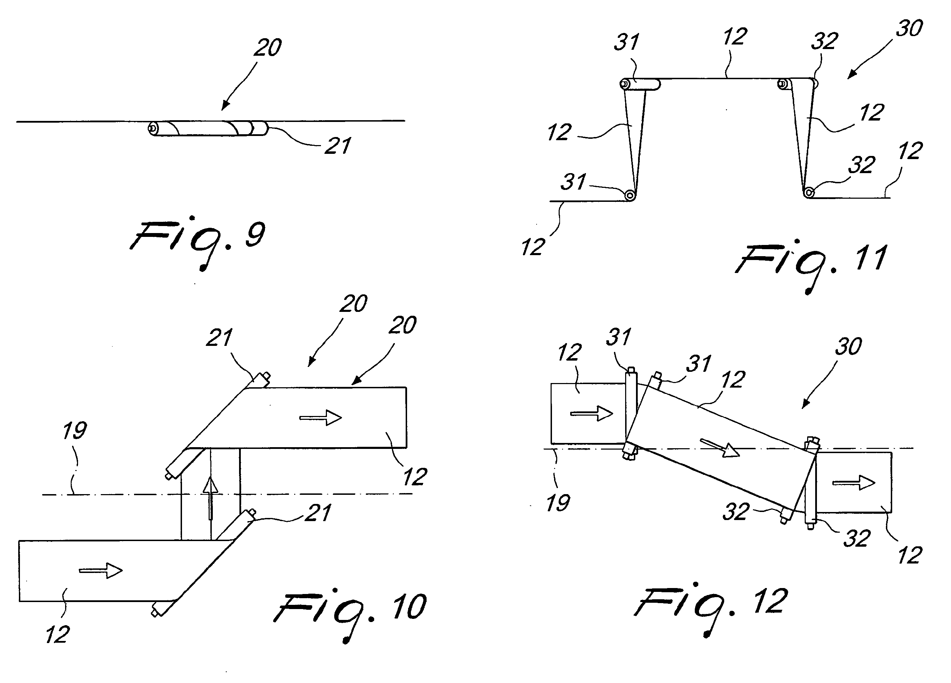

Figure 9 is a side view of an embodiment of the means for diverting the supporting fabric;
Figure 10 is a plan view of the diversion means of the preceding figure;
Figure 11 is a side view of another embodiment of the diversion means of the supporting fabric;
Figure 12 is a plan view of the diversion means of the preceding figure;
Figure 13 is a schematic view of an application system according to the prior art;
Figure 14 is a schematic view of another wing application system, according to the prior art;
Figure 15 is a schematic view of another wing application system, according to the prior art;
Figure 16 is a schematic view of another wing application system according to the prior art;
Figure 17 is a plan view of a chassis of a diaper with two elastic wings applied thereto;

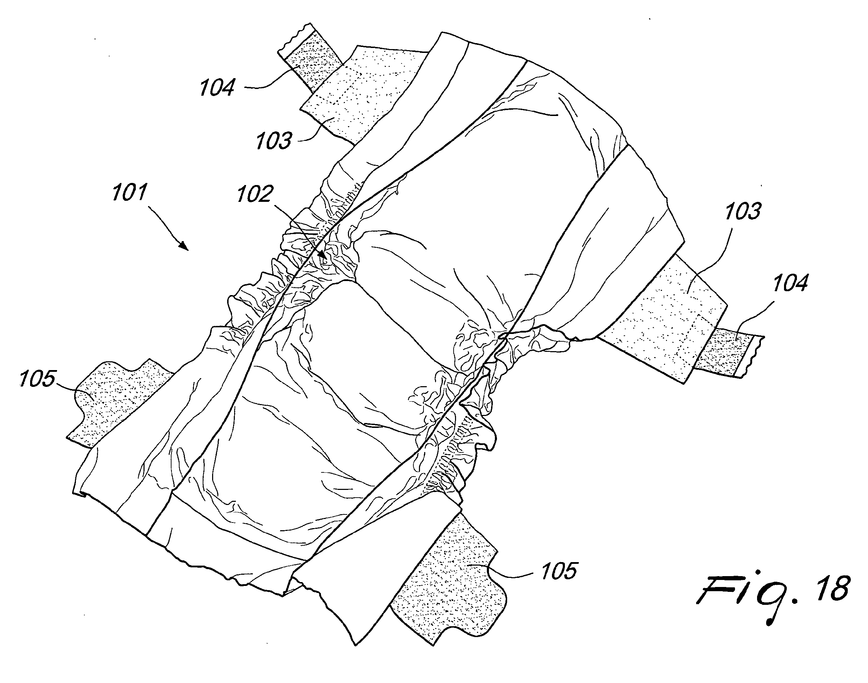

Figure 18 is a perspective view of a complete diaper, made with the method and the apparatus according to the present invention.

With reference to the cited figures, the method for the application of trapezoidal elastic wings to the chassis of a diaper, according to the invention, comprises providing a ribbon of elastic material 2 from which adjacent alternated trapezoidal panels, designated by the reference numeral 3, are obtained.

Each panel 3 has a smaller base 4 and a larger base 5, which are alternated on each side of the ribbon 2.

The trapezoidal panels are obtained by means of a single shaping device 7.

A tape 6 is applied to the smaller base 4 of each panel 3 by means of a single applicator for both sides of the ribbon 2. The applicator, which is not shown in the figures, is arranged upstream of the shaping device 7.

The tapes 6 are therefore applied to the ribbon 2 in positions that correspond, after passage through the shaping device 7, to the positions of the smaller bases 4 of each panel 3.

The trapezoidal panel 3 provided with the respective tape 6 constitutes an elastic wing 10.

It should be noted that an elastic wing can also be formed by means of panels made of non-elastic material associated with elastic tapes.

A support material ribbon 12, which will constitute the chassis of diaper, is taken by a first suction roller 8 at a first wing applicator 9, which is adapted to apply wings 10 to a first side 121 of the supporting ribbon 12.

After the application of the wings 10 to the first side 121, the supporting ribbon 12 is diverted axially and sent to a second suction roller 14, at the shaping device 7, where a second wing applicator 13 applies the wings 10 to the second side 122 of the support material ribbon 12.

The method according to the present invention therefore allows to use a single unwinder for the ribbon of elastic material 2, two unwinders for the tapes with a single applicator, a single functional unit that shapes the panels 3, and two applicators of the elastic wings that are fully independent.

The application of the elastic wings 10 to the two sides of the support material ribbon occurs in two separate and successive moments.

This is made possible after diverting the supporting fabric 12 from its trajectory along the production machine axis, first on one side and then on the other side.

The expression "machine axis" is understood to reference the imaginary line of the production machine that runs along the centerline of the diaper.

The apparatus according to the invention, generally designated by the reference numeral 1, comprises a single functional unit constituted by the shaping device 7 and by the applicators 9 and 13.

The shaping device 7 has a rotating suction blade 71. A contrast roller 72 acts on rotating suction blade 71.

A first binding roller 81 is arranged at the first suction roller 8 and is adapted to bind the wings 10 to the first side 121 of the supporting ribbon 12.

A second binding roller 141 is arranged at the second suction roller 14 and is adapted to bind the wings 10 on the second side 122 of the supporting ribbon 12.

The apparatus 1 comprises a diverting means for axially diverting the supporting ribbon 12. The diverting means comprises a roller assembly, or other adapted systems, capable of diverting the supporting ribbon 12 from its trajectory along the axis of the production machine, first on one side and then on the other side.

Figures 9 and 10 are views of an embodiment of an axial diversion means, generally designated by the reference numeral 20, which is constituted by a pair of mutually parallel oblique rollers 21.

Figures 11 and 12 are views of another preferred embodiment of an axial diversion means, generally designated by the reference numeral 30, which is constituted by two pair of repairs of oblique rollers, designated respectively by the reference numerals 31 and 32.

As is evident from the plan views of Figures 10 and 12, the axial diversion means allows to divert the ribbon 12 with respect to the axis 19 of the machine.

Figures 6-8 illustrate an embodiment of the apparatus which includes a first folding device 40 and a first binding device 41 for the wing 10 that is folded onto the first side 121 of the ribbon 12.

The first folding device 40 and the first binding device 41 are arranged downstream of the first suction roller 8 and of the corresponding first binding roller 81.

The apparatus also comprises a second folding device 50 and a second binding device 51 for the folded wing 10 onto the second side 122 of the ribbon 12.

The second folding device 50 and the second binding device 51 are arranged downstream of the second suction roller 14 and of the corresponding second binding roller 141.

In practice it has been found that the invention achieves the intended aim and objects, providing a method and an apparatus that allow to obtain a diaper with an elastic wing having an excellent aesthetic appearance, i.e., of the trapezoidal type, while avoiding waste of expensive raw material.

The method and the apparatus according to the present invention allow to vary the size of the diaper, in the same machine, in a simple and economic manner because it is possible to use a support material ribbon having a different width.

Another important advantage of the present invention is the simplicity of the method and of the apparatus and its ease of optimization.

This application claims the priority of Italian Patent Application No. MI2011A001956, filed on October 27, 2011.

## Claims

1. A method for applying trapezoidal elastic wings (10) to the chassis of a diaper, **characterized in that** it comprises the steps of:
providing a panels ribbon (2), from which alternating adjacent trapezoidal panels (3) are obtained, each having a smaller base (4) and a larger base (5) that are alternated on each side of said panels ribbon (2);
applying a tape (6) to said panels ribbon (2), at the smaller base (4) of each of said panels (3), and providing an elastic wing (10) constituted by said trapezoidal panel (3) provided with said respective tape (6);
applying said wings (10) to a first side (121) of a support material ribbon (12), by means of a single functional unit (7, 9, 13);
diverting said support material ribbon (12) and feeding again said support material ribbon (12) to said single functional unit (7, 9, 13);
applying said wings (10) to a second side (122) of said support material ribbon (12) by means of said single functional unit (7, 9, 13).

2. The method according to claim 1, **characterized in that** said trapezoidal panels (3) are provided by means of a single shaping device (7).

3. The method according to claim 2, **characterized in that** said tape (6) is applied by means of a single unit, for both sides of said panels ribbon (2), upstream of said shaping device (7); said tapes (6) being applied to said panels ribbon (2) in positions that will correspond, after passage through said shaping device (7), to the position of said smaller base (4) of each one of said panels (3).

4. An apparatus for applying trapezoidal elastic wings (10) to the chassis of a diaper, **characterized in that** its comprises a single functional unit constituted by a shaping device (7) and by a pair of wing applicators (9, 13); said single functional unit receiving a support material ribbon (12) and a panels ribbon (2); said support material ribbon (12) having a first side (121) and a second side (122); said panels ribbon (2) having first and second elastic wings (10) provided thereon; said single functional unit applying first elastic wings (10) to a first side (121) of said support material ribbon (12); said apparatus comprising an axial diversion means (20, 30); said axial diversion means (20, 30) receiving said support material ribbon (12), provided with first wings (10) applied to said first side (121), and feeding **again** said support material ribbon (12), provided with first wings (10) applied to said first side (121), to said single functional unit; said single functional unit applying said second wings (10) to said second side (122) of said support material ribbon (12), aligned with said first elastic wings (10).

5. The apparatus according to claim 4, **characterized in that** said shaping device (7) comprises a contrast roller (72) acting on a rotating suction blade (71) which cuts said panels ribbon (12), providing trapezoidal panels (3).

6. The apparatus according to claim 5, **characterized in that** said single functional unit comprises a first suction roller (8) which retains said support material ribbon (12) and said wings (10), and a first binding roller (9) which binds said first wings (10) to said first side (121) of said support material ribbon (12); said single functional unit further comprising a second suction roller (14) which retains said support material ribbon (12), that arrives from said diversion means (20, 30), and said first wings (10), and a second binding roller (13) which binds said second wings (10) to said second side (122) of said support material ribbon (12).

7. The apparatus according to claim 4, **characterized in that** said axial diversion means (20, 30) comprises a roller or belt system (21, 31, 32).

8. The apparatus according to claim 6, **characterized in that** it comprises a first folding device (40) and a first binding device (41) for binding a folded wing (10) to said first side (121) of said support material ribbon (12); said first folding device (40) and said first binding device (41) being arranged downstream of said first suction roller (8) and of said first binding roller (81); a second folding device (50) and a second binding device (51) for binding a folded wing (10) to said second side (122) of said support material ribbon (12); said second folding device (50) and said second binding device (51) being arranged downstream of said second suction roller (14) and of said second binding roller (141).

## Patentansprüche

1. Ein Verfahren zum Anbringen trapezförmiger elastischer Flügel (10) an den Rumpf einer Windel, **dadurch gekennzeichnet, dass** es folgende Schritte umfasst:
Bereitstellung eines Bandes von Panelen (2), aus dem alternierende benachoarte trapezförmige Panele (3) gewonnen werden, die jeweils eine kleinere Basis (4) und eine größere Basis (5) haben, die sich auf jeder Seite des Bandes von Panelen (2) abwechseln,
Anbringung eines Streifens (6) an dem Band von Panelen (2), an der kleineren Basis (4) jedes der Panele (3), und Bereitstellung eines elastischen Flügels (10), der aus dem trapezförmigen Panel (3), versehen mit dem entsprechenden Streifen (6), besteht,
Anbringung der Flügel (10) an einer ersten Seite (121) eines Trägermaterialbandes (12) mit Hilfe einer einzigen Funktionseinheit (7, 9, 13),
Umlenkung des Trägermaterialbandes (12) und erneute Zufuhr des Trägermaterialbandes (12) in die einzige Funktionseinheit (7, 9, 13),
Anbringung der Flügel (10) an einer zweiten Seite (122) des Trägermaterialbandes (12) mit Hilfe der einzigen Funktionseinheit (7, 9, 13).

2. Das Verfahren gemäß Anspruch 1, **dadurch gekennzeichnet, dass** die trapezförmigen Panele (3) mit Hilfe einer einzigen Formvorrichtung (7) bereitgestellt werden.

3. Das Verfahren gemäß Anspruch 2, **dadurch gekennzeichnet, dass** der Streifen (6) mit Hilfe einer einzeigen Einheit für beide Seiten des Bandes von Panelen (2), stromaufwärts von der Formvorrichtung (7), angebracht wird, wobei die Streifen (6) an dem Band von Panelen (2) in Positionen angebracht werden, die, nach dem Durchlauf durch die Formvorrichtung (7), der Position der kleineren Basis (4) jedes der Panele (3) entsprechen.

4. Eine Vorrichtung zur Anbringung trapezförmiger elastischer Flügel (10) am Rumpf einer Windel, **dadurch gekennzeichnet, dass** sie eine einzige Funktionseinheit umfasst, die aus einer Formvorrichtung (7) und einem Paar von Flügelapplikatoren (9, 13) besteht, wobei die einzige Funktionseinheit ein Trägermaterialband (12) und ein Band von Panelen (2) empfängt, wobei das Trägermaterialband (12) eine erste Seite (121) und eine zweite Seite (122) hat, wobei an das Band von Panelen (2) erste und zweite elastische Flügel (10) angebracht sind, wobei die einzige Funktionseinheit erste elastische Flügel (10) an einer ersten Seite (121) des Trägermaterialbandes (12) anbringt und die Vorrichtung ein axiales Umlenkmittel (20, 30) umfasst, wobei das axiale Umlenkmittel (20, 30) das Trägermaterialband (12), versehen mit ersten Flügeln (10), die an der ersten Seite (121) angebracht sind, empfängt und das Träaermaterialband (12), versehen mit ersten Flügeln (10), die an der ersten Seite (121) angebracht sind, erneut der einzigen Funktionseinheit zuführt, wobei die einzige Funktionseinheit die zweiten Flügel (10) an der zweiten Seite (122) des Trägermaterialbandes (12), in Ausrichtung mit den ersten elastischen Flügeln (10), anbringt.

5. Die Vorrichtung gemäß Anspruch 4, **dadurch gekennzeichnet, dass** die Formvorrichtung (7) eine Gegenwalze (72) umfasst, die auf eine sich drehende Saugrakel (71) einwirkt, welche das Band von Panelen (12) schneidet und trapezförmige Panele (3) liefert.

6. Die Vorrichtung gemäß Anspruch 5, **dadurch gekennzeichnet, dass** die einzige Funktionseinheit eine erste Saugwalze (8) umfasst, die das Trägermaterialband (12) und die Flügel (10) trägt, und eine erste Bindewalze (9), die die ersten Flügel (10) an die erste Seite (121) des Trägermaterialbandes (12) bindet, wobei die einzige Funktionseinheit weiter eine zweite Saugwalze (14) umfasst, die das Trägermaterialband (12) hält, das von dem Umlenkmittel (20, 30) ankommt, und die ersten Flügel (10), und eine zweite Binderolle (13), welche die zweiten Flügel (10) an die zweite Seite (122) des Trägermaterialbandes (12) bindet.

7. Die Vorrichtung gemäß Anspruch 4, **dadurch gekennzeichnet, dass** das axiale Umlenkmittel (20, 30) ein Walzen- oder Bandsystem (21, 31, 32) umfasst.

8. Die Vorrichtung gemäß Anspruch 6, **dadurch gekennzeichnet, dass** sie eine erste Faltvorrichtung (40) und eine erste Bindevorrichtung (41) zum Binden eines gefalteten Flügels (10) an die erste Seite (121) des Trägermaterialbandes (12) umfasst, wobei die erste Faltvorrichtung (40) und die erste Bindevorrichtung (41) stromabwärts von der ersten Saugwalze (8) und von der ersten Bindewalze (81) angeordnet sind; eine zweite Faltvorrichtung (50) und eine zweite Bindevorrichtung (51) zum Binden eines gefalteten Flügels (10) an die zweite Seite (122) des Trägermaterialbandes (12), wobei die zweite Faltvorrichtung (50) und die zweite Bindevorrichtung (51) stromabwärts von der zweiten Saugwalze (14) und von der zweiten Bindewalze (141) angeordnet sind.

## Revendications

1. Procédé destiné à l'application d'ailettes élastiques trapézoïdales (10) sur le châssis d'une couche-culotte, **caractérisé en ce qu'**il comprend les étapes :
de fournir un ruban de panneaux (2) duquel des panneaux trapézoïdaux adjacents alternants (3) sont obtenus, chacun ayant une base plus petite (4) et une base plus grande (5) qui sont en alternance sur chaque côté du ruban de panneaux (2);
d'appliquer un adhésif (6) sur le ruban de panneaux (2), sur la plus petite base (4) de chacun des panneaux (3) et de fournir une ailette élastique (10) constituée par le panneau trapézoïdal (3) doté de l'adhésif (6) respectif;
d'appliquer les ailettes (10) sur un premier côté (121) d'un ruban de matériau support (12), au moyen d'une unité fonctionnelle unique (7, 9, 13);
de dévier le ruban de matériau support (12) et d' alimenter à nouveau le ruban de matériau support (12) vers l'unité fonctionnelle unique (7, 9, 13);
d'appliquer les ailettes (10) sur un second côté (122) du ruban de matériau support (12) au moyen de l'unité fonctionnelle unique (7, 9, 13).

2. Procédé selon la revendication 1, **caractérisé en ce que** les panneaux trapézoïdaux (3) sont fournis via un dispositif de mise en forme (7) unique.

3. Procédé selon la revendication 2, **caractérisé en ce que** l'adhésif (6) est appliqué au moyen d'une unité unique, pour les deux cotés du ruban de panneaux (2), en amont du dispositif de mise en forme (7); les adhésifs (6) étant appliqués sur le ruban de panneaux (2) dans des positions qui vont correspondre, après le passage au travers du dispositif de mise en forme (7), à la position de la base plus petite (4) de chacun des panneaux (3).

4. Appareil pour l'application d'ailettes élastiques trapézoïdales (10) sur le châssis d'une couche-culotte, **caractérisé en ce qu'**il comprend une unité fonctionnelle unique constituée d'un dispositif de mise en forme (7) et d'une paire d'applicateurs d'ailettes (9, 13); l'unité fonctionnelle unique recevant un ruban de matériau support (12) et un ruban de panneaux (2); le ruban de matériau support (12) présentant un premier côté (121) et un second côté (122); le ruban de panneaux (2) présentant des première et secondes ailettes élastiques (10) prévues dessus; l'unité fonctionnelle unique appliquant les premières ailettes élastiques (10) sur un premier côté (121) du ruban de matériau support (12); l'appareil comprenant un moyen de déviation axiale (20, 30); le moyen de déviation axiale (20, 30) recevant le ruban de matériau support (12), doté de premières ailettes (10) appliquées sur le premier côté (121) et alimentant à nouveau le ruban de matériau support (12), doté de premières ailettes (10) appliquées sur le premier côté (121), vers l'unité fonctionnelle unique; l'unité fonctionnelle unique appliquant les secondes ailettes (10) sur le second côté (122) du ruban de matériau support (12), alignées avec les premières ailettes élastiques (10).

5. Appareil selon la revendication 4, **caractérisé en ce que** le dispositif de mise en forme (7) comprend un rouleau de contraste (72) agissant sur une lame d'aspiration rotative (71) qui coupe le ruban de panneaux (2), fournissant des panneaux trapézoïdaux (3).

6. Appareil selon la revendication 5, **caractérisé en ce que** l'unité fonctionnelle unique comprend un premier rouleau d'aspiration (8) qui retient le ruban de matériau support (12) et les ailettes (10), et un premier rouleau d'attache (9) qui attache les premières ailettes (10) au premier côté (121) du ruban de matériau support (12); l'unité fonctionnelle unique comprenant en outre un second rouleau d'aspiration (14) qui retient le ruban de matériau support (12)qui arrive du moyen de déviation (20, 30), et les premières ailettes (10), et un second rouleau d'attache (13) qui attache les secondes ailettes (10) au second côté (122) du ruban de matériau support (12).

7. Appareil selon la revendication 4, **caractérisé en ce que** le moyen de déviation axiale (20, 30) comprend un système de rouleau ou de courroie (21, 31, 32).

8. Appareil selon la revendication 6, **caractérisé en ce qu'**il comprend un premier dispositif de pliage (40) et un premier dispositif d'attache (41) pour attacher une ailette pliée (10) sur le premier côté (121) du ruban de matériau support (12); le premier dispositif de pliage (40) et le premier dispositif d'attache (41) étant agencés en aval du premier rouleau d'aspiration (8) et du premier rouleau d'attache (81); un second dispositif de pliage (50) et un second dispositif d'attache (51) pour attacher une ailette pliée (10) au second côté (122) du ruban de matériau support (12); le second dispositif de pliage (50) et le second dispositif d'attache (51) étant agencés en aval du second rouleau d'aspiration (14) et du second rouleau d'attache (141).
